# EUROPEAN PATENT SPECIFICATION

(11) **EP 3 870 143 B1**
(45) Date of publication and mention of the grant of the patent: **09.04.2025**
(21) Application number: 18816417.2
(22) Date of filing: 26.11.2018
(51) Int. Cl.: A61K 8/49, A61K 8/92, A61Q 19/10, A61Q 17/00

(54) **PERSONAL CARE COMPOSITIONS**
KÖRPERPFLEGE
COMPOSITIONS POUR LES SOINS PERSONNELS

(43) Date of publication of application: 01.09.2021
(73) Proprietor: Colgate-Palmolive Company, New York, NY 10022 (US)
(72) Inventor: WU, Qiang, Hillsborough, New Jersey 08844 (US); CHENG, Shujiang, Warren, New Jersey 07059 (US); DU-THUMM, Laurence, Princeton, New Jersey 08540 (US); ARMAS, Adriana, Ciudad de México, CP 11200 (MX); PARKER, Jodie, Parsippany, New Jersey 07054 (US); KOZUBAL, Cheryl, Somerset, New Jersey 08873 (US)
(74) Representative: Wichmann, Hendrik
(86) International application number: PCT/US2018/062411
(87) International publication number: WO 2020/112074

(56) References cited:
- EP-A1- 3 159 012
- WO-A1-99/27904
- US-A1- 2003 235 550
- US-A1- 2013 230 609

## Description

### BACKGROUND

Washing and cleansing skin with personal cleansing compositions (e.g., bar soaps, shower gels, body washes, cleansing lotions, liquid soaps, etc.) may often leave the skin feeling dry. For example, conventional liquid cleansing compositions may often include one or more surfactants, perfumes, preservatives, antimicrobial agents, and the like, that may strip moisture from the skin to thereby leave the skin feeling overly dry or chapped. In addition to over-drying skin, the increased use of conventional liquid cleansing compositions that incorporate antimicrobial agents may lead to antimicrobial resistance.

In view of the foregoing, moisturizers (e.g., emollients) may often be added to the cleansing compositions and/or lotions may be applied direct to the skin after washing to replenish, condition, and/or prevent excess dryness of the skin. Additionally, antimicrobial agents may often be omitted or utilized sparingly in the personal cleansing compositions. While the moisturizers may attempt to restore or replenish some of the moisture stripped from the skin by conventional cleansing compositions, the moisturizers will not treat the skin by reversing the damage already caused by the conventional cleansing compositions. For example, the moisturizers included in the liquid cleansing compositions do not promote or enhance the production of natural moisturizing factors (NMFs) in the skin to thereby treating or allowing the skin to regulate its hydration and repair any damage. Additionally, the removal of antimicrobial agents from personal cleansing compositions raises consumer concerns regarding the transmission of microbes.

What is needed, then, are improved personal cleansing compositions and methods for increasing the production of natural moisturizing factors and antimicrobial peptides in skin. Antimicrobial and/or moisturizing personal care compositions are described in US 2003/0235550 A1, WO 99/27904 A1 and US 2013/230609 A1. EP 3 159 012 A1 describes nanoemulsions which can be used in cosmetic products.

### BRIEF SUMMARY

This summary is intended merely to introduce a simplified summary of some aspects of one or more implementations of the present disclosure. Further areas of applicability of the present disclosure will become apparent from the detailed description provided hereinafter. This summary is not an extensive overview, nor is it intended to identify key or critical elements of the present teachings, nor to delineate the scope of the disclosure. Rather, its purpose is merely to present one or more concepts in simplified form as a prelude to the detailed description below.

The foregoing and/or other aspects and utilities embodied in the present disclosure may be achieved by providing a personal care composition which is a personal cleansing composition as defined in the claims and includes a solid carrier, one plant oil consisting of flaxseed oil or linseed oil, and one or more salts of pyrrolidone carboxylate.

In at least one implementation, the plant oil and the one or more salts of pyrrolidone carboxylate are each present in an effective amount to increase natural moisturizing factors (NMFs) in skin when applied to the skin.

In at least one implementation, the plant oil and the one or more salts of pyrrolidone carboxylate are each present in an effective amount to increase antimicrobial peptide (AMP) in skin when applied to the skin.

In at least one implementation, the plant oil is present in an amount of from about 0.01 weight % to about 5 weight %, about 0.5 weight % to about 4.5 weight %, or about 2 weight % to about 3 weight %, based on a total weight of the personal care composition.

In at least one implementation, the one or more salts of pyrrolidone carboxylate are present in an amount of from about 0.01 weight % to about 5 weight %, about 0.5 weight % to about 4.5 weight %, or about 2 weight % to about 3 weight %, based on a total weight of the personal care composition.

In at least one implementation, the plant oil consists of flaxseed oil.

The one or more salts of pyrrolidone carboxylate is selected from one or more of sodium pyrrolidone carboxylate, potassium pyrrolidone carboxylate, ammonium pyrrolidone carboxylate, or combinations thereof.

In at least one implementation, the one or more salts of pyrrolidone carboxylate comprises sodium pyrrolidone carboxylate, optionally, consists essentially of sodium pyrrolidone carboxylate, further optionally, consists of sodium pyrrolidone carboxylate.

In at least one implementation, the personal care composition is a bar soap.

The carrier is a solid carrier.

The foregoing and/or other aspects and utilities embodied in the present disclosure may be achieved by providing a method for preparing any one of the personal care composition disclosed herein, the method includes contacting the plant oil, the one or more salts of pyrrolidone carboxylate, and the carrier with one another.

Personal care compositions disclosed herein can be used in a method for treating microbes on skin, wherein the method includes contacting the skin with the personal care composition. In at least one implementation, contacting the skin increases production of antimicrobial peptides in or on the skin. In at least one implementation, contacting the skin with any one of the personal care compositions disclosed herein increases production of antimicrobial peptide biomarker LL-37 in or on the skin. In at least one implementation, contacting the skin with any one of the personal care compositions disclosed herein increases an innate immune response.

Personal care compositions disclosed herein can be used in a method for treating one or more dry skin conditions. The method includes contacting the skin with the personal care composition. In at least one implementation, contacting the skin with any one of the personal care compositions disclosed herein increases an amount of natural moisturizing factors in or on the skin. In at least one implementation, contacting the skin with any one of the personal care compositions disclosed herein increases an amount of Caspace-14 in or on the skin.

Further areas of applicability of the present disclosure will become apparent from the detailed description provided hereinafter. It should be understood that the detailed description and specific examples, while indicating some typical aspects of the disclosure, are intended for purposes of illustration only and are not intended to limit the scope of the disclosure.

### DETAILED DESCRIPTION

The following description of various typical aspect(s) is merely exemplary in nature and is in no way intended to limit the disclosure, its application, or uses.

As used throughout this disclosure, ranges are used as shorthand for describing each and every value that is within the range. It should be appreciated and understood that the description in a range format is merely for convenience and brevity, and should not be construed as an inflexible limitation on the scope of any embodiments or implementations disclosed herein. Accordingly, the disclosed range should be construed to have specifically disclosed all the possible subranges as well as individual numerical values within that range. As such, any value within the range may be selected as the terminus of the range. For example, description of a range such as from 1 to 5 should be considered to have specifically disclosed subranges such as from 1.5 to 3, from 1 to 4.5, from 2 to 5, from 3.1 to 5, etc., as well as individual numbers within that range, for example, 1, 2, 3, 3.2, 4, 5, etc. This applies regardless of the breadth of the range.

Unless otherwise specified, all percentages and amounts expressed herein and elsewhere in the specification should be understood to refer to percentages by weight. The amounts given are based on the active weight of the material.

Additionally, all numerical values are "about" or "approximately" the indicated value, and take into account experimental error and variations that would be expected by a person having ordinary skill in the art. It should be appreciated that all numerical values and ranges disclosed herein are approximate values and ranges, whether "about" is used in conjunction therewith. It should also be appreciated that the term "about," as used herein, in conjunction with a numeral refers to a value that may be ± 0.01% (inclusive), ± 0.1% (inclusive), ± 0.5% (inclusive), ± 1% (inclusive) of that numeral, ± 2% (inclusive) of that numeral, ± 3% (inclusive) of that numeral, ± 5% (inclusive) of that numeral, ± 10% (inclusive) of that numeral, or ± 15% (inclusive) of that numeral. It should further be appreciated that when a numerical range is disclosed herein, any numerical value falling within the range is also specifically disclosed.

As used herein, "free" or "substantially free" of a material may refer to a composition, component, or phase where the material is present in an amount of less than 10.0 weight %, less than 5.0 weight %, less than 3.0 weight %, less than 1.0 weight %, less than 0.1 weight %, less than 0.05 weight %, less than 0.01 weight %, less than 0.005 weight %, or less than 0.0001 weight % based on a total weight of the composition, component, or phase.

In the event of a conflict in a definition in the present disclosure and that of a cited reference, the present disclosure controls.

The present inventors have surprisingly and unexpectedly discovered that personal care products and personal care compositions thereof, including a carrier, one or more plant oils, and one or more salts of pyrrolidone carboxylate, significantly and unexpectedly increase the amount antimicrobial peptide (AMP) production in the skin. The present inventors have also surprisingly and expectedly discovered that personal care products and personal care compositions thereof, including a carrier, one or more plant oils, and one or more salts of pyrrolidone carboxylate, significantly and unexpectedly increase the amount of natural moisturizing factors (NMFs) in the skin.

### COMPOSITIONS

Compositions disclosed herein are personal care compositions, more precisely personal cleansing compositions as defined in the claims. As further described herein, the personal care compositions disclosed herein may be capable of or configured to facilitate, promote, enhance, or otherwise increase the production or formation of natural moisturizing factors (NMFs) in skin, thereby increasing hydration and barrier functions of the skin. As such, the personal care compositions disclosed herein may be utilized in the treatment of any one or more dry skin conditions. Illustrative dry skin conditions may be or include, but are not limited to, atoptic dermatitis, rosacea, psoriasis, or any combination thereof. The personal care compositions disclosed herein may also be capable of or configured to facilitate, promote, enhance, or otherwise increase the production or formation of antimicrobial peptides (AMPs) in skin, thereby providing protection on surfaces of the skin against one or more microbes and/or multicellular organisms (e.g., bacteria, viruses, etc.).

### Salts Of Pyrrolidone Carboxylate

The personal care composition include one or more salts of pyrrolidone carboxylate selected from sodium pyrrolidone carboxylate, potassium pyrrolidone carboxylate, ammonium pyrrolidone carboxylate, and combinations thereof. It should be appreciated that the L-isomer and/or the D-isomer may be utilized. For example, sodium D-pyrrolidone carboxylate, sodium L-pyrrolidone carboxylate, sodium DL-pyrrolidone carboxylate, potassium D-pyrrolidone carboxylate, potassium L-pyrrolidone carboxylate, potassium DL-pyrrolidone carboxylate, ammonium D-pyrrolidone carboxylate, ammonium L-pyrrolidone carboxylate, ammonium DL-pyrrolidone carboxylate, or any combination thereof may be utilized in the personal care composition. In an exemplary implementation, the one or more salts of pyrrolidone carboxylate includes sodium pyrrolidone carboxylate (NaPCA).

The one or more salts of pyrrolidone carboxylate may be present in the personal care composition in an effective amount or a therapeutically effective amount. As used herein, the expression or term "effective amount of one or more salts of pyrrolidone carboxylate," or the like, may refer to an amount of the one or more salts of pyrrolidone carboxylate sufficient to interact or work synergistically with the plant oil to elicit a response (e.g., biological, medical, etc.) of a tissue, system, animal, or human that is being sought. For example, the one or more salts of pyrrolidone carboxylate may be present in the personal care composition in an effective amount to interact or work synergistically with the plant oil to increase the production of NMFs and/or increase the production of antimicrobial peptides (AMPs) in skin.

In at least one implementation, the one or more salts of pyrrolidone carboxylate may be present in the personal care composition in an amount of from about 0.01 weight % to about 5 weight %, based on a total weight of the personal care composition. For example, the one or more salts of pyrrolidone carboxylate may be present in the personal care composition in an amount of from about 0.01 weight %, about 0.1 weight %, about 0.2 weight %, about 0.3 weight %, about 0.4 weight %, about 0.5 weight %, about 0.6 weight %, about 0.7 weight %, about 0.8 weight %, about 0.9 weight %, about 1 weight %, about 1.2 weight %, about 1.4 weight %, about 1.6 weight %, about 1.8 weight %, about 2 weight %, about 2.2 weight %, or about 2.4 weight % to about 2.6 weight %, about 2.8 weight %, about 3 weight %, about 3.2 weight %, about 3.4 weight %, about 3.6 weight %, about 3.8 weight %, about 4 weight %, about 4.1 weight %, about 4.2 weight %, about 4.3 weight %, about 4.4 weight %, about 4.5 weight %, about 4.6 weight %, about 4.7 weight %, about 4.8 weight %, about 4.9 weight %, or about 5 weight %, based on a total weight of the personal care composition. In another example, the one or more salts of pyrrolidone carboxylate may be present in the personal care composition in an amount of from about 0.01 weight % to about 5 weight %, about 0.1 weight % to about 4.9 weight %, about 0.2 weight % to about 4.8 weight %, about 0.3 weight % to about 4.7 weight %, about 0.4 weight % to about 4.6 weight %, about 0.5 weight % to about 4.5 weight %, about 0.6 weight % to about 4.4 weight %, about 0.7 weight % to about 4.3 weight %, about 0.8 weight % to about 4.2 weight %, about 0.9 weight % to about 4.1 weight %, about 1 weight % to about 4 weight %, about 1.2 weight % to about 3.8 weight %, about 1.4 weight % to about 3.6 weight %, about 1.6 weight % to about 3.4 weight %, about 1.8 weight % to about 3.2 weight %, about 2 weight % to about 3 weight %, about 2.2 weight % to about 2.8 weight %, or about 2.4 weight % to about 2.6 weight %, based on a total weight of the personal care composition. In yet another example, the one or more salts of pyrrolidone carboxylate may be present in the personal care composition in an amount of from greater than 0 weight %, greater than 0.01 weight %, greater than 0.1 weight %, greater than 0.2 weight %, greater than 0.3 weight %, greater than 0.4 weight %, greater than 0.5 weight %, greater than 0.6 greater than %, greater than 0.7 weight %, greater than 0.8 weight %, greater than 0.9 weight %, or greater than 1 weight %, based on a total weight of the personal care composition.

### Plant Oils

The personal care composition include one plant oil which consists of flaxseed oil or linseed oil. As used herein, "plant oil" may refer to oil that is obtained from a plant, or manufactured oil made by blending at least two components of oil (e.g., triglycerides, saturated and/or unsaturated fatty acids, etc.) to substantially mimic the composition of a natural plant oil or provide an oil substantially similar in composition to a plant oil. For example, a manufactured oil substantially similar in composition to a plant oil may include at least 50 weight %, at least 60 weight %, at least 70 weight %, at least 80 weight %, at least 90 weight %, at least 95 weight %, at least 98 weight %, at least 99 weight %, at least 99.5 weight %, at least 99.9 weight %, or 100 weight % of the components that are naturally found in the plant oil that the manufactured oil is designed to substantially mimic.

The plant oil may be present in the personal care composition in an effective amount or a therapeutically effective amount. As used herein, the expression or term "effective amount of one plant oil" or the like may refer to an amount of the oil sufficient to interact or work synergistically with the one or more salts of pyrrolidone carboxylate to elicit a response (e.g., biological, medical, etc.) of a tissue, system, animal, or human that is being sought. For example, the plant oil may be present in the personal care composition in an effective amount to interact or work synergistically with the one or more salts of pyrrolidone carboxylate to increase the production of NMFs and/or increase the production of antimicrobial peptides (AMPs) in skin.

The amount or concentration of the plant oil present in the solid cleansing composition may vary widely. In at least one implementation, the amount of the plant oil present in the personal care composition may be from greater than 0 weight % to less than or equal to 5 weight %, based on a total weight of the personal care composition. For example, the plant oil may be present in the personal care composition in an amount of from greater than 0 weight %, about 0.01 weight %, about 0.1 weight %, about 0.2 weight %, about 0.3 weight %, about 0.4 weight %, about 0.5 weight %, about 0.6 weight %, about 0.7 weight %, about 0.8 weight %, about 0.9 weight %, about 1 weight %, about 1.2 weight %, about 1.4 weight %, about 1.6 weight %, about 1.8 weight %, about 2 weight %, about 2.2 weight %, or about 2.4 weight % to about 2.6 weight %, about 2.8 weight %, about 3 weight %, about 3.2 weight %, about 3.4 weight %, about 3.6 weight %, about 3.8 weight %, about 4 weight %, about 4.1 weight %, about 4.2 weight %, about 4.3 weight %, about 4.4 weight %, about 4.5 weight %, about 4.6 weight %, about 4.7 weight %, about 4.8 weight %, about 4.9 weight %, or about 5 weight %, based on a total weight of the personal care composition. In another example, the plant oil may be present in the personal care composition in an amount of from greater than 0 weight %, about 0.01 weight % to about 5 weight %, about 0.1 weight % to about 4.9 weight %, about 0.2 weight % to about 4.8 weight %, about 0.3 weight % to about 4.7 weight %, about 0.4 weight % to about 4.6 weight %, about 0.5 weight % to about 4.5 weight %, about 0.6 weight % to about 4.4 weight %, about 0.7 weight % to about 4.3 weight %, about 0.8 weight % to about 4.2 weight %, about 0.9 weight % to about 4.1 weight %, about 1 weight % to about 4 weight %, about 1.2 weight % to about 3.8 weight %, about 1.4 weight % to about 3.6 weight %, about 1.6 weight % to about 3.4 weight %, about 1.8 weight % to about 3.2 weight %, about 2 weight % to about 3 weight %, about 2.2 weight % to about 2.8 weight %, or about 2.4 weight % to about 2.6 weight %, based on a total weight of the personal care composition. In yet another example, the plant oil may be present in the personal care composition in an amount of from greater than 0 weight %, greater than 0.01 weight %, greater than 0.1 weight %, greater than 0.2 weight %, greater than 0.3 weight %, greater than 0.4 weight %, greater than 0.5 weight %, greater than 0.6 greater than %, greater than 0.7 weight %, greater than 0.8 weight %, greater than 0.9 weight %, or greater than 1 weight %, based on a total weight of the personal care composition.

The plant oil and the one or more salts of pyrrolidone carboxylate may be present in an effective weight ratio or a therapeutically effective weight ratio to elicit a response (e.g., biological medical, etc.) of a tissue, system, animal, or human that is being sought. For example, the plant oils and the salts of pyrrolidone carboxylate may be present in a weight ratio sufficient to interact or work synergistically with one another to increase the production of NMFs and/or increase the production of antimicrobial peptides (AMPs) in skin.

In at least one implementation, the weight ratio of the plant oils to the salts of pyrrolidone carboxylate may be from about 0.5:1 to about 3.5:1. For example, the weight ratio of the plant oils to the salts of pyrrolidone carboxylate may be from about 0.5:1, about 0.6:1, about 0.7:1, about 0.9:1, about 1.0:1, about 1.1:1, about 1.2:1, about 1.3:1, about 1.4:1, about 1.5:1, about 1.6:1, about 1.7:1, about 1.8:1, about 1.9:1, about 2.0:1, about 2.1:1, or about 2.2:1 to about 2.3:1, about 2.4:1, about 2.5:1, about 2.6:1, about 2.7:1, about 2.8:1, about 2.9:1, about 3.0:1, about 3.1:1, about 3.2:1, about 3.3:1, about 3.4:1, or about 3.5:1. In another example, the weight ratio of the plant oils to the salts of pyrrolidone carboxylate may be from about 1.0:1 to about 3.5:1, about 1.1:1 to about 3.4:1, about 1.2:1 to about 3.3:1, about 1.3:1 to about 3.2:1, about 1.4:1 to about 3.1:1, about 1.5:1 to about 3.0:1, about 1.6:1 to about 2.9:1, about 1.7:1 to about 2.8:1, about 1.8:1 to about 2.7:1, about 1.9:1 to about 2.6:1, about 2.0:1 to about 2.5:1, about 2.1:1 to about 2.4:1, or about 2.2:1 to about 2.3:1. In a preferred implementation, the weight ratio of the plant oils to the salts of pyrrolidone carboxylate may be from about 2.0:1 to about 3:1, preferably about 2.2:1 to about 2.8:1, more preferably about 2.4:1 to about 2.6:1. In at least one exemplary implementation, the weight ratio of the plant oil to the one or more salts of pyrrolidone carboxylate may be about 2.5:1.

### Carrier or Excipient

The personal care composition may include the plant oil and the one or more salts of pyrrolidone carboxylate dispersed in, mixed with, dissolved in, combined with, or otherwise contacted with the solid carrier. In at least one implementation, the carrier may be capable of or configured to store, entrain, or otherwise contain the plant oils and the salts of pyrrolidone carboxylate, and deliver the plant oils and the salts of pyrrolidone carboxylate. It should be appreciated that the components or contents of the carrier and the respective amount of each of the components of the carrier may be at least partially determined by the type or use of the personal care product or the composition thereof. Illustrative personal care products or compositions thereof that may include the plant oil and the salts of pyrrolidone carboxylate may include, but are not limited to, body washes, soaps, including bar soaps, face washes, and shampoos,.

The personal care product or the composition thereof may be a personal hand and/or body cleansing composition. Illustrative personal hand and/or body cleansing compositions may include, but are not limited to, bar soaps, and body washes. The carrier for the personal hand and/or body cleansing composition or the personal hand and/or body conditioning composition may include, but is not limited to, any one or more of fragrances, essential oils, emulsifying agents, thickening agents, colorants, surfactants, natural actives, therapeutic actives, stain prevention actives, antimicrobial agents, vitamins, natural extracts, amino acids, enzymes and/or other proteins, abrasives, odor control agents, conditioning agents, moisturizers, humectants, occlusive agents, skin lipid fluidizers, lipophilic actives, hydrophilic materials, pearlizers, opacifying agents, and any mixture or combination thereof, in addition to any one or more of the other carrier components as discussed above.

The carrier may be hydrophilic or hydrophobic. The carrier may be anhydrous. The carrier is solid, particularly solid at room temperature.

Unless otherwise specifically identified, the ingredients for use in the compositions and formulations of the compositions disclosed herein are preferably cosmetically acceptable ingredients. As used herein, the expression "cosmetically acceptable" may refer to a component or ingredient that is suitable for use in a formulation for topical application to human skin. A cosmetically acceptable excipient, may refer to an excipient that is suitable for external application in the amounts and concentrations contemplated in the formulations of the compositions disclosed herein, and includes for example, excipients which are "Generally Recognized as Safe" (GRAS) by the United States Food and Drug Administration (FDA).

### METHODS

The present disclosure also provides a method for preparing a personal care composition thereof as defined in the claims. The method includes mixing, stirring, combining, or otherwise contacting the solid carrier, the plant oil, and the one or more salts of pyrrolidone carboxylate with one another. In another implementation, the method may include adding, mixing, stirring, combining, or otherwise contacting the plant oil and the one or more salts of pyrrolidone carboxylate with the solid carrier. In at least one example, the carrier is a base solid cleansing composition. For example, the carrier may be a bar soap.

The present disclosure also provides a personal care composition of the invention for use in methods for treating microbes on skin as defined in the claims. The method may include increasing production of AMPs in the skin by contacting any one or more of the personal care compositions disclosed herein with the skin. The method may also include reducing the amount of microbes on the skin by increasing the production of AMPs in the skin. The method may also include increasing production of AMP biomarker LL-37 in the skin. It should be appreciated that mammalian skin or dermis is constantly challenged by microbes but infections are minimized due to the cutaneous production of antimicrobial peptides (AMPs), which serve as one of the primary systems for protecting the skin. It should further be appreciated that LL-37 is one of the peptide forms of cathelicidin, the first AMP discovered in mammalian skin. Cathelicidin dysfunction is a central factor in the pathogenesis of several cutaneous conditions and/or diseases. For example, in atopic dermatitis, cathelicidin is suppressed; in rosacea, cathelicidin peptides are abnormally processed to forms that induce inflammation; and in psoriasis, cathelicidin peptides convert self-DNA to potent stimulus in an auto inflammatory cascade. As such, it should be appreciated that the increased production of the AMP biomarker LL-37 in the skin may treat, reduce, or ameliorate one or more skin conditions or diseases, such as atopic dermatitis, rosacea, and psoriasis, or one or more symptoms thereof.

The method for treating microbes on skin may also include enhancing or increasing the innate immune response by treating the skin with any one or more of the personal care compositions disclosed herein. The one or more microbes may be or include, but are not limited to, gram negative bacteria, gram positive bacteria, any strains that are resistant to conventional antibiotics, mycobacteria, enveloped viruses, fungi, transformed and/or cancerous cells, other microbes, or any combination thereof.

The present disclosure furthers provide a personal care composition of the invention for use in methods for treating one or more dry skin conditions as defined in the claims. Illustrative dry skin conditions may be or include, but are not limited to, atopic dermatitis, rosacea, and psoriasis. It should be appreciated that atopic dermatitis may result in a deficiency of filaggrin (filament-aggregating protein), a protein at least partially responsible for skin barrier functions and NMF production. It should further be appreciated that filaggrin contributes to the physical strength of the stratum corneum (SC) barrier through its integral involvement in the filament matrix complex in the inner layer of the stratum corneum (SC). In the outer layer of the SC, filaggrin is degraded into NMFs. It should be appreciated that NMFs are integral to the function of the SC as NMFs provide moisture retention (humectancy), maintain the acidic pH and buffering capacity of the SC, promote proper epidermal maturation and desquamation, and decrease pathogenic bacterial colonization. The method for treating one or more skin conditions may include increasing amounts of natural moisturizing factors (NMFs) in skin by contacting any one or more of the personal care compositions disclosed herein with the skin. The method may include increasing production or amount of a Caspace-14 gene in the skin to promote filaggrin degradation to thereby produce NMF in the skin.

The present disclosure may further provide a personal care composition of the invention for use in a method of making a personal care composition for treating microbes on skin and/or treating one or more dry skin conditions. The method may include combining or otherwise contacting one plant oil and one or more salts of pyrrolidone carboxylate with a carrier to prepare the personal care composition.

The present disclosure may also provide a personal care composition of the invention for use in methods of cleansing skin and/or enhancing hydration and barrier functions of the skin in a patient in need thereof via the enhanced production of Caspase-14 and/or NMF. Patients in need thereof may have relatively lower natural moisturizing factors (NMFs) and/or relatively low amounts or concentration of Caspase-14 in the skin, which may be evidenced by dry and/or chapped skin. The method may include providing an effective amount of the personal cleansing composition to enhance Caspase-14 and/or NMF in skin, contacting the personal cleansing composition to the skin or hair, and optionally, rinsing the personal cleansing composition from the skin or hair with water. In at least one embodiment, the personal cleansing composition may be combined with added water prior to or while contacting the personal cleansing composition with the skin or hair.

### EXAMPLES

The examples and other implementations described herein are exemplary and not intended to be limiting in describing the full scope of compositions, medical uses and methods of this disclosure, which is defined in the claims.

### Example 1

A base solid cleansing composition was prepared. A control solid cleansing composition (1) and three test solid cleansing compositions (2)-(4) were then prepared by adding varying amounts of flaxseed oil (FSO) and sodium PCA (NaPCA) to the base solid cleansing composition according to Table 1. Each of the solid cleansing compositions (1)-(4) were tested as 1% soap samples. Particularly, each of the solid cleansing compositions (1)-(4) were diluted in water in a weight ratio of 1:20, and subsequently diluted in phosphate-buffered saline (PBS) in a weight ratio of 1:5. Cleansing composition (4) is an example of a composition according to the present invention. Cleansing compositions (1)-(3) are comparative examples.

**Table 1**

| **Compositions of Control and Test Solid Cleansing Compositions (1) - (5)** | | | |
|---|---|---|---|
| **#** | **Amount of Base Solid Cleansing Composition (wt%)** | **Amount of Flaxseed Oil (wt%)** | **Amount of Sodium PCA (wt%)** |
| **1** | 100 | 0 | 0 |
| **2** | 99.5 | 0.5 | 0 |
| **3** | 99.9 | 0 | 0.1 |
| **4** | 99.4 | 0.5 | 0.1 |

### Example 2

Each of the control and test solid cleansing compositions (1)-(4) was evaluated *in vitro* on skin tissue models to observe the production of antimicrobial peptides (AMP), particularly, AMP LL-37. 3D human skin models obtained from MatTek Corp. of Ashland, MA, were utilized as the models in the *in vitro* study, and LL-37 production was monitored with an ELISA Kit. To conduct the *in vitro* study, 30 µm of respective 1% solutions of the control solid cleansing composition (1) or one of the test solid cleansing compositions (2)-(5) was topically applied to respective 3D human skin models and incubated at about 37°C for about 1 hour. After about 1 hour, each of the 3D human skin models was thoroughly and gently washed with PBS about 5 to about 8 times. Each of the 3D human skin models was then placed in fresh media and incubated at about 37°C for about 24 hours. The 3D human skin models were then collected and lysed with Heat Shock Protein (HSP) lysis buffer, and the tissue was broken down by exposing the tissue to a shaker operating at the speed of 15.01/s for 15 minutes twice. After lysing with the HSP lysis buffer, each of the lysed samples were frozen or maintained at about - 80°C. The production of AMP biomarker LL-37, as measured by the ELISA kit, from respective human skin models treated with each of the control and test solid cleansing compositions (1)-(4) is summarized in Table 2. All measurements were done in triplicate, averaged, and normalized to the total protein in each of the 3d human skin models.

**Table 2**

| **Amount of LL-37 Measured from Skin Models Treated with 1% Solutions of Control and Test Solid Cleansing Compositions (1)-(4)** | | | | | |
|---|---|---|---|---|---|
| # | **Amount of Flaxseed Oil (wt%)** | **Amount of Sodium PCA (wt%)** | **Amount of LL-37** | **Std Dev** | **Change from Control** |
| 1 | 0 | 0 | 8.28 | 2.63 | -- |
| 2 | 0.5 | 0 | 6.63 | 0.28 | -1.65 |
| 3 | 0 | 0.1 | 7.87 | 1.32 | -0.41 |
| 4 | 0.5 | 0.1 | 8.80 | 2.27 | 0.52 |

As illustrated in Table 2, the test solid cleaning composition (2) including only flaxseed oil exhibited a decrease in LL-37. Similarly, the test solid cleansing composition (3) including only sodium PCA exhibited a decrease in LL-37. However, surprising and unexpectedly, the synergistic combination of sodium PCA and flaxseed oil in test solid cleansing composition (4) exhibited an increase in LL-37 when a decease was expected. Specifically, it was not expected that combining the sodium PCA, which resulted in a decrease in LL-37, with flaxseed oil, which also resulted in a decrease in LL-37, would result in a significant increase in the amount of LL-37 measured in the skin models, as demonstrated in the test solid cleansing composition (4). It should be appreciated that the increase in biomarker LL-37 indicates the increase of antimicrobial peptide (AMP) production.

### Example 3 (comparative example)

A base liquid cleansing composition (i.e., shower gel) was prepared. A control liquid cleansing composition (5) and three test liquid cleansing compositions (6)-(8) were then prepared by adding varying amounts of flaxseed oil (FSO) and sodium PCA (NaPCA) to the base liquid cleansing composition according to Table 3. Each of the liquid cleansing compositions (5)-(8) were tested as 2% gel samples. Particularly, each of the liquid cleansing compositions (5)-(8) were diluted in water in a weight ratio of 1:10, and subsequently diluted in phosphate-buffered saline (PBS) in a weight ratio of 1:5.

**Table 3**

| **Com positions of Control and Test Liquid Cleansing Compositions (1) - (5)** | | | |
|---|---|---|---|
| **#** | **Amount of Base Liquid Cleansing Composition (wt%)** | **Amount of Flaxseed Oil (wt%)** | **Amount of Sodium PCA (wt%)** |
| **5** | 100 | 0 | 0 |
| **6** | 99.5 | 0.5 | 0 |
| **7** | 99.9 | 0 | 0.1 |
| **8** | 99.4 | 0.5 | 0.1 |

### Example 4 (comparative example)

Each of the control and test liquid cleansing compositions (5)-(8) was evaluated *in vitro* on skin tissue models to observe the production of natural moisturizing factors (NMFs), particularly, Caspase-14. 3D human skin models obtained from MatTek Corp. of Ashland, MA, were utilized as the models in the *in vitro* study, and the Caspase-14 production was monitored with an ELISA Kit. To conduct the *in vitro* study, 30 µm of respective 2% solutions of the control liquid cleansing composition (5) or one of the test liquid cleansing compositions (6)-(8) was topically applied to respective 3D human skin models and incubated at about 37°C for about 1 hour. After about 1 hour, each of the 3D human skin models were thoroughly and gently washed with PBS about 5 to about 8 times. Each of the 3D human skin models was then placed in fresh media and incubated at about 37°C for about 24 hours. The 3D human skin models were then collected and lysed with (Heat Shock Protein) lysis buffer, and the 3D human skin models were broken down with a shaker at a speed of about 15.01/s for 15 minutes twice. After lysing with the HSP lysis buffer, each of the lysed samples were frozen or maintained at about -80°C. The production of NMF biomarker Caspase-14, as measured by the ELISA kit, from respective human skin models treated with each of the control and test liquid cleansing compositions (5)-(8) is summarized in Table 4. All measurements were done in triplicate, averaged, and normalized to the total protein in each of the 3D human skin models.

**Table 4**

| **Amount of Caspase-14 Measured from Skin Models Treated with 2% Solutions of Control and Test Liquid Cleansing Compositions (5)-(8)** | | | | | |
|---|---|---|---|---|---|
| # | **Amount of Flaxseed Oil (wt%)** | **Amount of Sodium PCA (wt%)** | **Amount of Caspase-14** | **Std Dev** | **Change from Control** |
| 5 | 0 | 0 | 41.93 | 0.94 | -- |
| 6 | 0.5 | 0 | 47.85 | 13.79 | 5.92 |
| 7 | 0 | 0.1 | 37.46 | 7.31 | -4.47 |
| 8 | 0.5 | 0.1 | 53.21 | 3.96 | 11.28 |

As illustrated in Table 4, the test liquid cleaning composition (6) including only flaxseed oil exhibited an increase in Caspace-14, and the test liquid cleansing composition (7) including only sodium PCA exhibited a decrease in Caspace-14. However, surprising and unexpectedly, the synergistic combination of sodium PCA and flaxseed oil in test liquid cleansing composition (8) exhibited a significant increase in Caspace-14 when a decrease was expected. Specifically, it was not expected that combining the sodium PCA, which resulted in a decrease in Caspace-14, with flaxseed oil, which resulted in an increase in Caspace-14, would result in a significant increase in the amount of Caspace-14 measured in the skin models, as demonstrated in the test liquid cleansing composition (8). It should be appreciated that the increased amount of biomarker Caspase-14 indicates the production of NMF, as Caspace-14 is utilized in the degradation of filaggrin to produce NMF in skin.

## Claims

1. A personal cleansing composition, comprising:
a carrier; wherein the carrier is a solid carrier;
one plant oil; wherein the one plant oil consists of flaxseed oil or linseed oil; and
one or more salts of pyrrolidone carboxylate wherein the one or more salts of pyrrolidone carboxylate is selected from sodium pyrrolidone carboxylate, potassium pyrrolidone carboxylate, ammonium pyrrolidone carboxylate, and combinations thereof.

2. The personal cleansing composition of claim 1, wherein the one plant oil and the one or more salts of pyrrolidone carboxylate are each present in an amount of from 0.01 weight % to 5 weight %, based on a total weight of the personal cleansing composition.

3. The personal cleansing composition of any one of the preceding claims, wherein the one plant oil is present in an amount of from 0.5 weight % to 4.5 weight %, or 2 weight % to 3 weight %, based on a total weight of the personal cleansing composition.

4. The personal cleansing composition of any one of the preceding claims, wherein the one or more salts of pyrrolidone carboxylate are present in an amount of from 0.5 weight % to 4.5 weight %, or 2 weight % to 3 weight %, based on a total weight of the personal cleansing composition.

5. The personal cleansing composition of any one of the preceding claims, wherein the one or more salts of pyrrolidone carboxylate consists of sodium pyrrolidone carboxylate.

6. The personal cleansing composition of any one of the preceding claims, wherein the personal cleansing composition is a bar soap.

7. A method for preparing the personal cleansing composition of any one of the preceding claims, the method comprising contacting the one plant oil, the one or more salts of pyrrolidone carboxylate, and the carrier with one another.

8. A personal cleansing composition of any one of claims 1-6 for use in a method for treating microbes on skin, said use comprising contacting the skin with the personal cleansing composition.

9. A personal cleansing composition of any one of claims 1-6 for use in a method for treating one or more dry skin conditions, the method comprising contacting the skin with the personal cleansing composition.

10. The personal cleansing composition for use according to claim 9, wherein said use includes increasing an amount of natural moisturizing factors in or on the skin.

11. The personal cleansing composition for use according to claim 8 or claim 9, wherein said use includes increasing an amount of Caspace-14 in or on the skin.

## Patentansprüche

1. Körperreinigungszusammensetzung, umfassend:
einen Träger; wobei der Träger ein fester Träger ist;
ein Pflanzenöl, wobei das eine Pflanzenöl aus Leinsamenöl oder Leinöl besteht, und
ein oder mehrere Salze des Pyrrolidoncarboxylats, wobei das eine oder die mehreren Salze des Pyrrolidoncarboxylats ausgewählt sind unter Natriumpyrrolidoncarboxylat, Kaliumpyrrolidoncarboxylat, Ammoniumpyrrolidoncarboxylat und Kombinationen davon.

2. Körperreinigungszusammensetzung nach Anspruch 1, wobei das eine Pflanzenöl und das eine oder die mehreren Salze des Pyrrolidoncarboxylats jeweils in einer Menge von 0,01 Gew.-% bis 5 Gew.-%, bezogen auf das Gesamtgewicht der Körperreinigungszusammensetzung, vorhanden sind.

3. Körperreinigungszusammensetzung nach einem der vorhergehenden Ansprüche, wobei das eine Pflanzenöl in einer Menge von 0,5 Gew.-% bis 4,5 Gew.-% oder 2 Gew.-% bis 3 Gew.-%, bezogen auf das Gesamtgewicht der Körperreinigungszusammensetzung, vorhanden ist.

4. Körperreinigungszusammensetzung nach einem der vorhergehenden Ansprüche, wobei das eine oder die mehreren Salze des Pyrrolidoncarboxylats in einer Menge von 0,5 Gew.-% bis 4,5 Gew.-% oder 2 Gew.-% bis 3 Gew.-%, bezogen auf das Gesamtgewicht der Körperreinigungszusammensetzung, vorhanden sind.

5. Körperreinigungszusammensetzung nach einem der vorhergehenden Ansprüche, wobei das eine oder die mehreren Salze des Pyrrolidoncarboxylats aus Natriumpyrrolidoncarboxylat besteht/bestehen.

6. Körperreinigungszusammensetzung nach einem der vorhergehenden Ansprüche, wobei die Körperreinigungszusammensetzung eine Stückseife ist.

7. Verfahren zur Herstellung der Körperreinigungszusammensetzung nach einem der vorhergehenden Ansprüche, wobei das Verfahren ein Inkontaktbringen des einen Pflanzenöls, des einen oder der mehreren Salze des Pyrrolidoncarboxylats und des Trägers miteinander umfasst.

8. Körperreinigungszusammensetzung nach einem der Ansprüche 1 bis 6 zur Verwendung in einem Verfahren zur Behandlung von Mikroben auf der Haut, wobei die Verwendung ein Inkontaktbringen der Haut mit der Körperreinigungszusammensetzung umfasst.

9. Körperreinigungszusammensetzung nach einem der Ansprüche 1 bis 6 zur Verwendung in einem Verfahren zur Behandlung eines oder mehrerer trockener Hautzustände, wobei das Verfahren ein Inkontaktbringen der Haut mit der Körperreinigungszusammensetzung umfasst.

10. Körperreinigungszusammensetzung zur Verwendung gemäß Anspruch 9, wobei die Verwendung die Erhöhung einer Menge natürlicher Feuchthaltefaktoren in oder auf der Haut einschließt.

11. Körperreinigungszusammensetzung zur Verwendung gemäß Anspruch 8 oder Anspruch 9, wobei die Verwendung die Erhöhung einer Menge von Caspace-14 in oder auf der Haut einschließt.

## Revendications

1. Composition d'hygiène personnelle, comprenant :
un support ; le support étant un support solide ;
une huile végétale ; l'huile végétale étant constituée d'huile de graines de lin ou d'huile de lin ; et
un ou plusieurs sels de pyrrolidone carboxylate, lesdits un ou plusieurs sels de pyrrolidone carboxylate étant choisis parmi le pyrrolidone carboxylate de sodium, le pyrrolidone carboxylate de potassium, le pyrrolidone carboxylate d'ammonium et les combinaisons de ceux-ci.

2. Composition d'hygiène personnelle selon la revendication 1, dans laquelle l'huile végétale et lesdits un ou plusieurs sels de pyrrolidone carboxylate sont chacun présents en une quantité de 0,01 % en poids à 5 % en poids, par rapport au poids total de la composition d'hygiène personnelle.

3. Composition d'hygiène personnelle selon l'une quelconque des revendications précédentes, dans laquelle l'huile végétale est présente en une quantité de 0,5 % en poids à 4,5 % en poids, ou de 2 % en poids à 3 % en poids, par rapport au poids total de la composition d'hygiène personnelle.

4. Composition d'hygiène personnelle selon l'une quelconque des revendications précédentes, dans laquelle lesdits un ou plusieurs sels de pyrrolidone carboxylate sont présents en une quantité de 0,5 % en poids à 4,5 % en poids, ou de 2 % en poids à 3 % en poids, par rapport au poids total de la composition d'hygiène personnelle.

5. Composition d'hygiène personnelle selon l'une quelconque des revendications précédentes, dans laquelle lesdits un ou plusieurs sels de pyrrolidone carboxylate sont constitués de pyrrolidone carboxylate de sodium.

6. Composition d'hygiène personnelle selon l'une quelconque des revendications précédentes, dans laquelle la composition d'hygiène personnelle est un pain de savon.

7. Procédé de préparation de la composition d'hygiène personnelle selon l'une quelconque des revendications précédentes, le procédé comprenant la mise en contact de l'huile végétale, du ou des sels de pyrrolidone carboxylate et du support les uns avec les autres.

8. Composition d'hygiène personnelle selon l'une quelconque des revendications 1 à 6 pour une utilisation dans une méthode de traitement de microbes sur la peau, ladite utilisation comprenant la mise en contact de la peau avec la composition d'hygiène personnelle.

9. Composition d'hygiène personnelle selon l'une quelconque des revendications 1 à 6 pour une utilisation dans une méthode de traitement d'un ou plusieurs états de sécheresse cutanée, la méthode comprenant la mise en contact de la peau avec la composition d'hygiène personnelle.

10. Composition d'hygiène personnelle pour une utilisation selon la revendication 9, ladite utilisation comprenant l'augmentation d'une quantité de facteurs hydratants naturels dans ou sur la peau.

11. Composition d'hygiène personnelle pour une utilisation selon la revendication 8 ou la revendication 9, dans laquelle ladite utilisation comprend l'augmentation d'une quantité de Caspace-14 dans ou sur la peau.
